(19) Europäisches Patentamt
European Patent Office
Office européen des brevets

(11) **EP 4 640 152 A1**

(12) **EUROPEAN PATENT APPLICATION**

(43) Date of publication:
**29.10.2025 Bulletin 2025/44**

(21) Application number: **24172484.8**

(22) Date of filing: **25.04.2024**

(51) International Patent Classification (IPC):
**A61B 5/327** $^{(2021.01)}$    **A61B 5/00** $^{(2006.01)}$

(52) Cooperative Patent Classification (CPC):
**A61B 5/327; A61B 5/7217;** A61B 5/7225;
A61B 5/7239

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **FRANCK, Christoph Florian**
**Eindhoven (NL)**

(74) Representative: **Philips Intellectual Property &
Standards
High Tech Campus 52
5656 AG Eindhoven (NL)**

(54) **PACEMAKER PULSE DETECTION**

(57)    A method for pacemaker pulse detection in ECG which is based on constructing an artificial ECG lead signal from recorded ECG lead signals or lead vectors using a weighted sum of lead signals with weights optimized such that a pre-defined objective function correlated with the strength of the pacemaker pulse signal is maximized, and then performing pace pulse detection within this artificial lead signal.

FIG. 2

Processed by Luminess, 75001 PARIS (FR)

**Description**

FIELD OF THE INVENTION

This invention relates to the field of pacemaker pulse detection in ECG signal data.

BACKGROUND OF THE INVENTION

Electrocardiography (ECG) is used for diagnosing heart conditions and for continuous monitoring of heart function. An ECG recorder measures small voltage signals (e.g. on the order of millivolts) occurring between points on the body surface of a patient as a result of physiological activity of the heart. ECG recorders may be provided as stand-alone devices, e.g. cardiographs, or integrated into multi-parameter patient monitors, e.g. Philips Intellivue family, or in the form of wearable devices, e.g. Holter monitors or smartwatches.

ECGs are commonly acquired by placing adhesive electrodes at defined locations on the body surface of a patient. The standard electrode locations are as follows:

Limb electrodes: LA - left arm, RA - right arm, LL - left leg
Precordial electrodes:V1: 4th intercostal space at the right sternal border; V2: 4th intercostal space at the left sternal border; V3: Between V2 and V4; V4: 5th intercostal space at the midclavicular line; V5: Level with V4 at the anterior axillary line; V6: Level with V5 at the midaxillary line.

From the electric potentials present at the electrode locations, standard lead vectors are computed based on (possibly weighted) potential differences between defined combinations of the electrodes. The standard lead vectors are outlined in table 1 below: (note that the notation Vn is standardly used to designate both lead vectors and electrode positions):

*Table 1*

| Lead | Type | Calculation |
|------|------|-------------|
| I | Bipolar Limb | $I = LA - RA$ |
| II | Bipolar Limb | $II = LL - RA$ |
| III | Bipolar Limb | $III = LL - LA$ |
| aVR | Augmented Unipolar Limb | $aVR = RA - \frac{1}{2}(LA + LL)$ |
| aVL | Augmented Unipolar Limb | $aVL = LA - \frac{1}{2}(RA + LL)$ |
| aVF | Augmented Unipolar Limb | $aVF = LL - \frac{1}{2}(RA + LA)$ |
| V1 | Precordial (Chest) | $V1 - \frac{1}{3}(RA + LA + LL)$ |
| V2 | Precordial (Chest) | $V2 - \frac{1}{3}(RA + LA + LL)$ |
| V3 | Precordial (Chest) | $V3 - \frac{1}{3}(RA + LA + LL)$ |
| V4 | Precordial (Chest) | $V4 - \frac{1}{3}(RA + LA + LL)$ |
| V5 | Precordial (Chest) | $V5 - \frac{1}{3}(RA + LA + LL)$ |
| V6 | Precordial (Chest) | $V6 - \frac{1}{3}(RA + LA + LL)$ |

Here, the value $\frac{1}{3}(RA + LA + LL)$ is known as the Wilson's central terminal. It is sometimes omitted in calculations of the precordial leads.

Furthermore, other electrode placement systems exist, such as EASI, which require fewer electrodes, and involve computational reconstruction of the standard lead vectors.

The standard 12-lead ECG consists of 12 lead vectors, which provide different views of the heart's electrical activity. These leads are divided into limb lead vectors (six in total) and precordial (chest) lead vectors (six in total). The limb lead vectors record the electrical activity in the frontal plane. The precordial lead vectors record the electrical activity in the horizontal plane.

In terms of directionality the limb lead vector direction may be summarized as follows.

Bipolar Limb Leads (3 Leads):

Lead I: Measures the voltage between the left arm and the right arm.
Lead II: Measures the voltage between the left leg and the right arm.
Lead III: Measures the voltage between the left leg and the left arm.

Augmented Unipolar Limb Leads (3 Leads):

aVR (augmented Vector Right): Measures the voltage at the right arm, with the other limbs forming the reference.
aVL (augmented Vector Left): Measures the voltage at the left arm, with the other limbs forming the reference.
aVF (augmented Vector Foot): Measures the voltage at the left leg (or foot), with the other limbs forming the reference.

In terms of directionality, the precordial (Chest) lead vectors may be summarized as follows:

V1: Voltage between the fourth intercostal space at the right sternal border and the heart.
V2: Voltage between the fourth intercostal space at the left sternal border and the heart.
V3: Voltage between the midway between positions for V2 and V4 and the heart.
V4: Voltage between the fifth intercostal space at the midclavicular line and the heart.
V5: Voltage between the horizontal to V4 at the left anterior axillary line and the heart.
V6: Voltage between the horizontal to V5 at the left midaxillary line and the heart.

The six standard limb lead vectors (I, II, III, aVR, aVL, aVF) cover the possible directions across the upper torso in steps of 30°.

While it is possible to calculate and use other, nonstandard lead vectors, e.g. LL-(1/3 RA + 2/3 * LA, or V1 - V6), this is not common practice for clinical use, as physicians are trained to evaluate ECG recordings that use the standard lead vectors.

Generally, lead vectors can be formed from the set of single-ended electrode voltage signals by means of a linear combination, where the sum of the linear combination coefficients is zero and the sum of the absolute values of the linear combination coefficients is two.

The voltage reproduction of each lead vector is most sensitive to voltage changes that occur parallel to the line between the electrode location (or virtual electrode locations) used in the relevant lead vector. The sensitivity, or apparent amplitude, of a voltage change decreases if the lead vector is not aligned with the direction of the voltage change. If a voltage change occurs at a 90° angle (i.e. perpendicular or orthogonal) to the direction of the lead vector, no voltage change is registered along this vector at all.

The degree of reproduction of a voltage change in a given lead vector depends therefore upon the angle $\alpha$ between the lead vector and the voltage change.

While modeling the pacemaker electrodes as an electrical dipole is complex, the relationship between the angle $\alpha$ and apparent, measured voltage can be approximated by the cosine of $\alpha$:

$$U\_apparent = U cos(\alpha)$$

In terms of vectors, this approximation can also be expressed as the scalar product of the voltage vector u with a unity-length vector indicating the lead vector direction $\underline{v}$:

$$U\_apparent = <\underline{v},\underline{u}> = \underline{v}^T\underline{u}.$$

where $<\underline{v}, \underline{u}>$ represents the scalar product between the vectors $\underline{v}$ and $\underline{u}$.

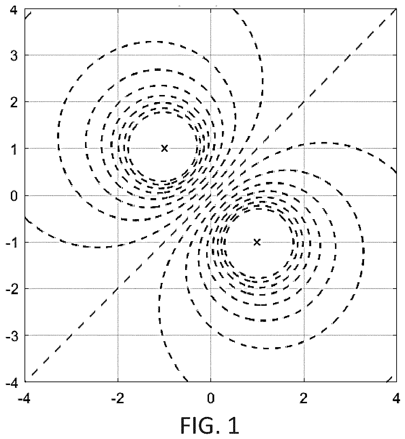

Fig. 1 shows the electric potential lines of an electric dipole consisting of two opposite point charges. The X and Y axes are the horizontal and vertical lines, respectively, that form the coordinate system in which the electric potential is mapped. Each point (x, y) in this system corresponds to a position in space relative to the dipole. The dashed lines represent equipotential lines; lines in space along which the electric potential is constant. In the context of an electric dipole, these lines are symmetrical about the line bisecting the two charges. An electric dipole consists of two point charges of equal magnitude but opposite sign. The two 'x' marks represent these point charges. The equipotential lines form patterns that are perpendicular to the electric field lines, where the electric field lines (not shown) radiate out from the positive charge and terminate at the negative charge. The electric field lines are perpendicular to the equipotential lines at every point. There is no potential difference between points on the same dashed line, hence zero voltage is measurable between points on the dashed diagonal line that is perpendicular to the line connecting the two point charges. The largest potential difference would be measured between points on the line containing the two point sources.

Fig. 1 illustrates how the orientation of the equipotential lines changes in a predictable way relative to the dipole axis and is always perpendicular to the direction of the electric field lines emanating from the positive to the negative charge.

The considerations above apply to any difference in electric potential, regardless of whether it is produced by the heart or by any other source, including a pacemaker.

Currently, pacemaker pulse detection in ECG recorders commonly uses one or more of the standard lead vectors. If only a subset of the standard lead vectors are used, the subset may be configurable by the user, or implicitly chosen for example by "primary lead" and "secondary lead" settings of the ECG recorder. An ECG recorder may run a pace pulse detection algorithm on all standard leads simultaneously. However, this is computationally expensive and entails redundant computations.

Pace pulse detection is commonly performed using a sampling rate that is substantially higher than the output sampling rate used for displaying and analyzing the ECG signal, since pace pulses are short, high-frequency events with durations between 0.04 and 2.0 milliseconds. An ECG recorder may run a pace pulse detection at a sampling rate of, for example, 4 kHz, 8 kHz or a higher frequency, while providing wave output at 500 Hz to 1000 Hz. The high sampling rate and complexity of searching for pace pulses with a range of possible duration and shape parameters make pace pulse detection algorithms computationally expensive.

In general, pacemaker pulses are distinguished from physiological signals by a larger slew rate. Pacemaker pulses can be distinguished from noise and interference also by a larger slew rate. Furthermore, pacemaker pulses are in general isolated, non-continuous events, as compared with interference which generally exhibits a degree of continuity. The angle at which a pacemaker pulse amplitude and slew rate is reproduced in a particular ECG lead vector directly affects the probability of detection the pulse in the presence of noise.

It is in general of advantage to minimize the duration and amplitude of pace pulses generated by pacemakers. In an optimal scenario, the pulses are just large enough to trigger a heart action ("capture") and no larger. Pulses with large amplitudes and long durations are a drain of battery charge and incur greater wear on the pacemaker device. The latter has the effect of requiring more frequent surgery to replace the pacemaker.

Some pacemakers include functionality for dynamically adapting the pulse parameters to the lowest-energy setting that still guarantees capture.

However, low amplitude pulses are more difficult to detect in the ECG recording, especially in the presence of noise.

The aim of maximizing battery lifetime of pacemakers has resulted in a general reduction in pace pulse amplitude in state-of-the-art pacemaker devices. However, this has had the consequent effect of reducing reliability of pace pulse detection by ECG recorders.

If an ECG recorder fails to detect pacemaker pulses that are present in the ECG signal, any subsequent analysis of the ECG waves be inaccurate. A heart rhythm which is normal for a patient who has a pacemaker may be misclassified as an unpaced, abnormal rhythm if the patient is assumed not to have a pacemaker. The ECG recorder may subsequently emit a false alarm. This may result in unnecessary treatment of the patient.

The direction along which a pacemaker pulse is applied is not necessarily identical to the direction of one of the standard lead vectors. As a result, even if an ECG recorder analyses all six standard limb leads for the presence of pace pulses, there may still be a misalignment of up to 15° with respect to the direction of pacemaker pulse injection. This translates to a reduction of the apparent pacemaker pulse amplitude and slope of 3.4%.

While this example loss is small and thus not typically significant, a significantly worse loss can arise if only two lead vectors are used for pacemaker pulse detection. For example, a worst case arises where vectors III and aVL are analyzed and pace pulse injection occurs along the direction of lead II. Here, the misalignment is 60° and the reduction of the apparent pulse amplitude and slope is 50%. In a further example case, two lead vectors may be used which are orthogonal in directionality. In this case, the misalignment can still be up to 45°, resulting in a reduction of the apparent pulse amplitude by 29%. Such reductions are significant if the pacemaker pulses are small enough to be close to the detection threshold of the ECG recorder.

The preceding discussion has considered only the two-dimensional case in which both ECG and pacemaker electrode are assumed to be located in the same, frontal plane. ECG chest electrodes are offset from this plane in the anteroposterior direction and add a third dimension to the ECG recording. The lines between pacemaker electrodes may be at an angle to the frontal plane, which leads to an additional possibility for misalignment and reduction of the apparent amplitude.

The possible misalignment between pacemaker pulse injection direction and the standard ECG lead vectors used for pace pulse detection result in reduced apparent amplitude of the pacemaker pulses. The fewer ECG lead vectors that are used to detect pace pulses, the larger the possible misalignment. It would be of advantage therefore to improve reliability of pacemaker pulse detection, such that in cases where pace pulse amplitude is small, pulse detection is not compromised.

In this regard, it is a further aim to improve pacemaker pulse detection reliability without significantly increasing computational demand, since this incurs greater power consumption. Analyzing as many standard lead vectors as possible for the presence of pacemaker pulses may provide fairly comprehensive detection, but it is not economical in terms of computational resources.

It is a further aim that the reliability of the pace pulse detection be independent of the particular setup of the ECG recorder, e.g. independent of user settings such as "primary lead" and "secondary lead" setting.

SUMMARY OF THE INVENTION

The invention is defined by the claims.

According to examples in accordance with an aspect of the invention, there is provided a method for detecting pacemaker pulses in electrocardiogram, ECG, data. The method comprises receiving ECG data comprising a plurality of recorded ECG lead signals, each recorded lead signal corresponding to a recording for a respective ECG lead of an ECG measurement apparatus.

The method further comprises constructing a synthetic lead signal from a weighted sum of at least a subset of the recorded lead signals and/or one or more lead vectors computed from at least a subset of the recorded ECG lead signals. In this context a lead vector comprises a linear combination of two or more of the recorded lead signals.

The method further comprises applying a pacemaker pulse signal detection algorithm to the synthetic pulse signal to detect and extract a pacemaker pulse signal from the synthetic lead signal.

The step of constructing a synthetic lead signal may comprise running an optimization procedure comprising identifying a set of weightings for the weighted sum which maximizes a value of a pre-defined objective function. The pre-defined objective function may be correlated with a strength of the pacemaker pulse signal in the synthetic lead signal.

The method thus comprises using the standard lead voltages and and/or standard lead vectors as a set of basis vectors and constructing a synthetic lead signal or vector as a weighted sum of the basis vectors which maximizes the strength of the pacemaker pulse signal in the resulting synthetic lead vector.

The effect of this is to effectively find a synthetic lead vector which has a direction which is aligned with a direction of the pacemaker pulse signal and thus maximizes the signal of the pacemaker pulse in the ECG data.

Preferably, the plurality of recorded ECG lead signals includes at least three recorded ECG lead signals, for example at least three single-ended input channels (electrodes). A setup with only two electrodes may only allow for one lead vector to be formed and thus have no adjustable directional parameters.

In some embodiments, the objective function includes at least a component which evaluates a maximum slew rate of the synthetic lead signal, or is indicative of / correlated with maximum slew rate. As a general rule, pacemaker pulses can be distinguished from physiological signals by their larger slew rates. Pacemaker pulses can be distinguished from noise and interference also by their larger slew rates. Thus, maximizing the maximum slew rate of a sample synthetic lead signal as part of the objective function help identify the synthetic lead signal with the strongest pace pulse signal.

In some embodiments, the objective function includes at least a component which evaluates a maximum gradient of the synthetic lead signal or is indicative of / correlated with the maximum gradient. Computing a quantitative value for the gradient is one way of targeting the slew rate of the signal.

In some embodiments, the objective function, when applied to a synthetic lead signal, results in: high pass filtering the synthetic lead signal or (e.g. numerically) differentiating the synthetic lead signal to yield a processed synthetic lead signal. The effect of high pass filtering the signal is to select for the higher frequency pace pulse signal components as opposed to the lower frequency physiological signal components. The effect of applying differentiation to the synthetic lead signal would be to highlight regions of the signal where there are rapid changes in amplitude, which again selects for the higher frequency pacemaker pulse signal components.

The objective function may further result in, subsequent to high pass filtering or numerically differentiating, computing a L-infinity norm of (e.g. a vector of samples of) the processed synthetic lead signal. The L-infinity Norm (also known as the Chebyshev norm or maximum norm) of a signal is a measure of the maximum absolute value of its elements (samples).

After the synthetic lead signal has been processed through high pass filtering or numerical differentiation (or both), computing the L-infinity norm of this processed signal yields a single scalar value. This value represents the maximum absolute change (in the case of numerical differentiation) or the maximum amplitude of the high frequency part (in the case of high pass filtering) of the signal. It is thus a measure of the extremeness or the peak values in the processed signal. The combined effect of these operations is to process the synthetic lead signal in a way that emphasizes high-frequency components or rapid changes (thus selecting for the pacemaker pulse parts of the signal) and quantifies the degree or magnitude of these features using the L-infinity norm.

In some embodiments, the optimization procedure is performed under a constraint that a sum of the weightings of the weighted sum is zero, i.e. $\mathbf{1}^T \cdot \mathbf{x} = 0$. In some embodiments, the optimization procedure is performed under a constraint that a sum of absolute values (L1-norm) of the weightings is less than or equal to two.

In some embodiments, the optimization procedure is performed under a constraint that a sum of absolute values (L1-norm) of the weightings is equal to two.

As noted above, one way of characterizing a lead vector is with the constraint that a sum of the linear combination coefficients of the individual lead signals forming the vector is zero and the sum of the absolute values of the linear combination coefficients is two. Thus, one or both of these constraints applied to the optimization helps in identifying a synthetic lead signal which conforms with optimal technical characteristics for a lead vector. Since the objective function is to be maximized, the L1-norm equality constraint may instead be relaxed to a less-or-equal constraint without affecting the optimal point.

In some embodiments, the objective function includes a pulse signal strength component correlated with an estimated strength of the pace pulse signal in the synthetic lead signal, and a noise cost component subtracted from the signal strength component, the noise cost component correlated with an estimated noise level in the synthetic lead signal. This allows for noise to be factored into the optimization.

There are different particular ways of determining the synthetic lead signal.

According to a first set of embodiments (referred to herein as 'Method 1') the synthetic lead signal may be constructed only from a weighted combination of the recorded lead signals. This represents an advantageously simple approach.

According to a further set of embodiments (referred to herein as 'Method 2'), the synthetic lead signal may be constructed from a weighted combination of measured lead vectors formed from the measured lead signals, and wherein the measured lead signals used in the weighted combination are orthogonal to one another. By forming the synthetic lead signal from a basis of two or three orthogonal basis lead vectors, the optimization is somewhat simplified (since only two vectors need to be included in the weighted combination which is being optimized).

Thus, in accordance with some embodiments, the method comprises computing from the recorded ECG lead signals a first lead vector and a second lead vector, and wherein the synthetic lead signal is constructed from a weighted combination of at least the first and second lead vectors.

In some embodiments, the first and second lead vectors are orthogonal in directionality.

More than two lead vectors may be used in the weighted combination.

The lead vectors used in the weighted combination may be a selection of two or more of the standard lead vectors (see table 1 above).

In some embodiments, the optimization procedure is performed under the constraint that the L2 norm of a vector of the weightings is less than or equal to 1.

In some embodiments, the optimization procedure is performed under the constraint that the L2 norm of a vector of the weightings is equal to 1.

In some embodiments, the optimization procedure is performed under the constraint that the L2 norm of a vector of the weightings is between 1 and $\sqrt{2}$ .

With regards to the pace pulse detection, in some embodiments, the pacemaker pulse detection algorithm comprises recurrently comparing samples of the synthetic lead signal against a trailing edge detection threshold and/or a leading edge detection threshold. The threshold(s) may be static or dynamic. A pacemaker pulse can in general be distinguished from a physiological pulse by the steepness of the trailing or leading edge.

A further aspect of the invention is a computer program product comprising computer program code configured, when run on a processor, to cause the processor to perform a method in accordance with any embodiment in this disclosure or any claim of this application.

A further aspect of the invention is a processing device, comprising one or more processors configured to perform a method in accordance with any embodiment described in this disclosure or any claim of this application.

A further aspect of the invention is a system comprising: a processing device in accordance with the above; and an ECG apparatus operatively coupled with the processing device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates electric potential lines of an electric dipole consisting of two opposite point charges;
Fig. 2 outlines steps of an example method in accordance with one or more embodiments of the invention;
Fig. 3 is a block diagram of an example processing device and system in accordance with one or more embodiments of the invention;
Fig. 4 illustrates example virtual electrode locations corresponding to an example synthetic lead signal;

Fig. 5 illustrates pace pulse detection probability as a function of pace pulse injection angle where one measured lead signal is used for the detection;

Fig. 6 illustrates pace pulse detection probability as a function of pace pulse injection angle where two measured lead signals are used;

Fig. 7 illustrates pace pulse detection probability as a function of pace pulse injection angle where a full set of measured lead signals are used; and

Fig. 8 illustrates pace pulse detection probability as a function of pace pulse injection angle where a synthetic lead signal determined according to embodiments of the present invention is used.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for pacemaker pulse detection in ECG which is based on constructing an artificial ECG lead signal from measured ECG lead signals or lead vectors in which the pacemaker pulse signal is maximized, and then performing pace pulse detection within this artificial lead signal.

Embodiments presented herein provide a method which is able to achieve optimal pacemaker pulse detection with a minimal number of detection channels. Embodiments involve first determining one or more ECG lead vectors, which may be synthetic (non-standard) lead vectors, and which are determined to be more optimally aligned with possible pace pulses than any other lead vectors. The ECG recorder then performs the pace pulse detection algorithm on the ECG signal of these one or more optimally aligned lead vectors. This maximizes the detection probability while keeping the number of channels used for pace pulse detection as small as possible.

By way of example, at least a subset of embodiments of the invention may include one or more of the following features:

an ECG measurement apparatus ('ECG recorder') with at least three input electrodes and pace pulse detection functionality;

pace pulse detection functionality performed by

first determining a (possibly synthetic) lead vector which is aligned with the direction of pace pulse injection and which produces a signal with optimal reproduction of the pacemaker pulse signal; and

identifying pacemaker pulses in the signal corresponding to the optimal lead vector. The identification uses known properties of pacemaker pulses, such as a threshold slew rate (e.g. using a trailing/leading edge detection threshold) and/or a defined range of pulse durations.

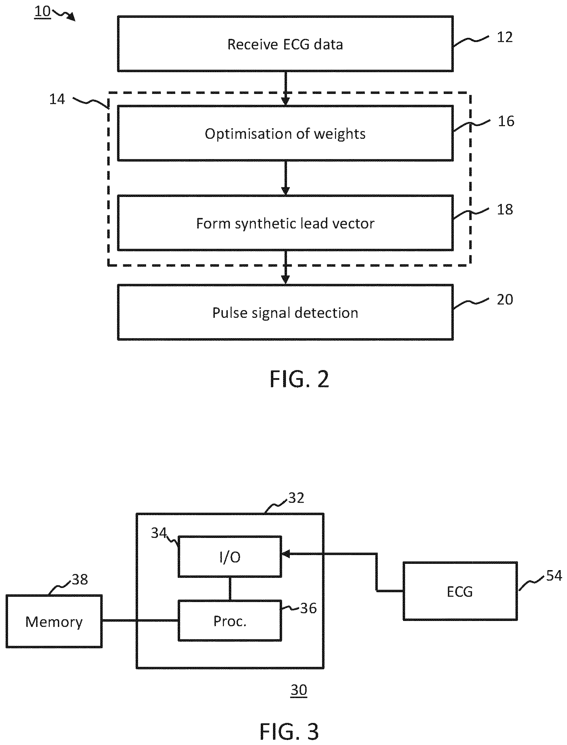

Fig. 2 outlines in block diagram form steps of an example method according to one or more embodiments. The steps will be recited in summary, before being explained further in the form of example embodiments.

Provided is a method 10 for detecting pacemaker pulses in electrocardiogram, ECG.

The method 10 comprises receiving 12 ECG data comprising a plurality of recorded ECG lead signals. Each recorded lead signal corresponds to a recording for a respective ECG lead of an ECG measurement apparatus.

The method 10 further comprises constructing 14 a synthetic lead signal from a weighted sum of at least a subset of the recorded lead signals and/or one or more lead vectors computed from at least a subset of the recorded ECG lead signals. In this context, a lead vector may be understood as comprising a linear combination of two or more of the recorded lead signals.

The constructing 14 of the synthetic lead signal comprises running an optimization procedure 16 comprising identifying a set of weightings for the weighted sum which maximizes a value of a pre-defined objective function. The pre-defined objective function is correlated with a strength of the pacemaker pulse signal in the synthetic lead signal. Once the weightings are determined, the synthetic lead signal is formed 18 using a linear combination of the measured ECG lead signals or lead vectors, according to the weightings determined in the optimization.

The method 10 further comprises applying 20 a pacemaker pulse signal detection algorithm to the synthetic pulse signal to detect and extract a pacemaker pulse signal from the synthetic lead signal.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises an ECG measurement apparatus 54 (otherwise known as an ECG recorder). For example, this may be a 12-lead ECG measurement apparatus.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

The effect of the optimization procedure is effectively to find a lead vector which is optimally aligned with the direction of pacemaker pulse injection. This therefore maximizes the strength of the pacemaker pulse signal in the lead signal/vector.

According to one set of embodiments, this can be achieved by seeking the synthetic lead signal (set of weighting coefficients for the weighted sum) which maximizes the maximum gradient or change or fluctuation within the signal of the synthetic lead vector voltage as a function of time. However, other approaches are also possible.

Each of the plurality of lead signals (or channels) may be recorded over a common time window.

The recorded lead signals, i.e. the input channel samples, might be represented in a matrix $\mathbf{A}$ which is formed from the vertical stacking of the individual lead signals, represented as vectors, $\mathbf{a}(n)$, where n indexes sample number.

The basic optimization procedure might then be expressed as

$$\text{maximize } f(\mathbf{A} \cdot \mathbf{x})$$

where $f(z)$ is the objective function and x represents a vector of weightings for the lead signals.

The optimization procedure may comprise evaluating the optimization function for each of a series of candidate sets of weightings, x, and iteratively maximizing the objective function.

The synthetic lead signal may be understood as a synthetic lead recording or a synthetic lead vector.

The ECG data preferably includes recordings for at least 3 single-ended ECG leads.

With regards to the process of determining the optimal dynamic lead vector, there are at least three approaches to achieving this, which may be referred to respectively as 'Method 1', 'Method 2', and 'Method 3'.

By way of summary, the first approach (Method 1) involves forming a synthetic lead signal from the measured lead signals, i.e. the single-ended electrode potential signals. This is as opposed to forming lead vectors from the measured lead signals and determining an optimal weighted combination of the lead vectors. The method involves, via the optimization, searching for the weighting coefficients, $\mathbf{x}$, of the optimal synthetic lead signal. This may be performed in some embodiments under one or both of the constraints that the sum of the weighting coefficients, $\mathbf{1}^T\mathbf{x}$, equals zero and/or the sum of the absolute values of the weighting coefficients (i.e. the L1 norm), $\|\mathbf{x}\|_1$, equals two (this definition describes vectors according to Einthoven's triangle and using linear interpolation between the electrode potentials).

The second approach (Method 2) involves forming a synthetic lead signal from a weighted sum of two or more measured lead vectors where each measured lead vector is formed from a linear combination of measured lead signals. For example, the measured lead vectors in this case could be two or more of the standard ECG lead vectors (see discussed earlier for further details). By way of just one example, two standard ECG lead vectors could be used which are orthogonal, e.g. lead I and lead aVF. The optimization procedure may then comprise searching for optimal weighting coefficients x for forming a weighted sum of these two measured lead vectors. This may be performed in some embodiments under the constraint that $\|\mathbf{x}\|_2 = 1$ (i.e. the L2 norm is equal to one). In the case where precordial electrodes are used, a precordial vector that is approximately orthogonal to the frontal plane may for example be used in addition to two orthogonal standard ECG lead vectors consisting of limb electrode potentials only.

The third approach (Method 3) involves forming a synthetic lead signal from a weighted sum of two or more measured lead vectors, but wherein the chosen measured lead vectors are arbitrary in the sense that they may be non-orthogonal. The measured lead vectors may be two or more of the standard ECG lead vectors. The optimization in this case may again be performed under the constraint that $\|\mathbf{x}\|_2 = 1$ (i.e. the L2 norm is equal to one).

Method 2 may be preferable over Method 1 since arbitrary standard lead vectors are not guaranteed to form an orthogonal basis. This may lead to apparent pace pulse amplitudes which are greater than the true amplitude. Method 2 may also be preferable to Method 3 for the same reason.

Regardless of the method used, there is a need to define the criteria for optimality of the synthetic lead signal/vector. In other words, there is a need to define the criteria which will be evaluated by the objective (or utility) function of the optimization.
This will now be discussed.

As mentioned previously, pacemaker pulses are characterized by slew rates that are significantly greater than those of physiological signals. Slew rates can be approximated from a digital signal by numeric differentiation, or by applying a high-pass filter with an appropriate corner frequency.

Accordingly, in some embodiments, the objective function may include at least a component which evaluates a maximum slew rate of the synthetic lead signal or which is at least indicative of or correlated with maximum slew rate. Additionally or alternatively, the objective function may include at least a component which evaluates a maximum gradient of the synthetic lead signal or which is indicative of or correlated with maximum gradient.

A class of objective/utility function which might be used is the L-infinity norm of the vector of output samples after numerical differentiation or high pass filtering. The L-infinity norm corresponds to the largest absolute value in the vector of output samples. In other words, in some embodiments, the objective function, when applied to a synthetic lead signal, results in: high pass filtering the synthetic lead signal or numerically differentiating the synthetic lead signal to yield a processed synthetic lead signal; and subsequently computing a L-infinity norm of the processed synthetic lead signal.

The optimization problem preferably may also apply one or more constraints that ensure that the final result has the typical technical characteristics of a lead vector. For example, lead vectors may be characterized by having input channel weighting coefficients that sum to zero. By way of further example, lead vectors may be characterized by having input channel weighting coefficients whose L1-norm is equal to two.

The measured ECG lead signals (i.e. the input channel samples) at the sampling instant $n$ may be collected in vectors, $\mathbf{a}(n)$, e.g. row vectors, e.g. $\mathbf{a}(n)=\mathbf{a}_n=(RA(n), LA(n), LL(n), V(n))$. The vectors of measured ECG lead signal samples $\mathbf{a}(n)$ may be stacked vertically to form the matrix of input sample values $\mathbf{A}$. Here, each different row of $\mathbf{A}$ corresponds to a different sample point, n, while each column of A corresponds to a different one of the measured ECG lead signals (i.e. the input channel samples). When $\mathbf{A}$ is vector multiplied with the vector $\mathbf{x}$ of weighting coefficients, this gives an output vector representing sample values of the synthetic lead signal, $\mathbf{s} = \mathbf{A} \cdot \mathbf{x}$. Here, s is a column vector, wherein each different element of $\mathbf{s}$ corresponds to the value for the synthetic lead signal at a different sample point, n.

The basic optimization problem to find x is

$$\text{maximize } f(\mathbf{A} \cdot \mathbf{x})$$

where f(z) is the objective function and $\mathbf{x}$ represents a vector of weightings for the lead signals.

For example, the optimization function, f(z), may to take the form:

$$f(\mathbf{s}) = f(\mathbf{A} \cdot \mathbf{x}) = \|g(\mathbf{A} \cdot \mathbf{x})\|_\infty$$

where g(z) represents a function whose output is the numerical differentiation or high pass filtered version of the input, z. This thus represents the L-infinity norm of the synthetic lead vector after numerical differentiation or high pass filtering.

In some embodiments, this may be applied with the following further constraints:

$$\mathbf{1}^{\mathrm{T}} \cdot \mathbf{x} = 0$$

$$\|\mathbf{x}\|_1 = 2$$

In other words, wherein the sum of the weighting coefficients, **x,** is zero and the L1-norm of the weighting coefficients is equal to two.

In this form, the optimization is a mixed-integer linear program ("MILP"),. The "mixed-integer" part is made necessary by maximizing an L-infinity norm and by equality-constraining an L1-norm. A Mixed-Integer Linear Program (MILP) is a type of optimization problem in which the goal is to find the best solution from a set of available solutions, based on a set of criteria.

'Mixed-Integer' refers to the fact that some of the decision variables are required to be integers (or binary), while others may be continuous. This mix of integer and continuous variables results in a "mixed-integer" optimization problem.

'Linear Program' refers to the fact that the relationships between the variables are linear. This means that the objective function and the constraints are linear equations or inequalities.

Since the aim of the optimization is to maximize the objective/utility function, the L1-norm equality constraint may be relaxed to a less-or-equal constraint without affecting the optimal point. In other words, the optimization might be modified to:

$$\text{maximize } f(\mathbf{A} \cdot \mathbf{x})$$

where

$$f(\mathbf{A} \cdot \mathbf{x}) = \|g(\mathbf{A} \cdot \mathbf{x})\|_\infty$$

where $g(z)$ represents a function whose output is the numerical differentiation or high pass filtered version of the input, z, and under the constraints:

$$\mathbf{1}^T \cdot \mathbf{x} = 0$$

$$\|\mathbf{x}\|_1 \leq 2$$

This optimization remains a MILP, but it is "mixed-integer" only due to the maximization of an L-infinity norm.

With regards to the matrix, $\mathbf{A}$, of input sample values, according to Method 1, this may be a matrix of sample values, $\mathbf{a}_n$, for two or more measured ECG lead signals, i.e. the measured signals for two or more single-ended electrodes, and for a plurality of sample points, n. According to Method 2, the matrix, $\mathbf{A}$, of input sample values may be a matrix of sample values, $\mathbf{a}_n$, for two or more measured ECG lead vectors for a plurality of sample points, n, meaning lead vectors formed from a linear combination of two or more of the measured lead signals and wherein the measured lead vectors are orthogonal. According to Method 3, the matrix, $\mathbf{A}$, of input sample values may be a matrix of sample values, $\mathbf{a}_n$, for two or more measured ECG lead vectors for a plurality of sample points, n, meaning lead vectors formed from a linear combination of two or more of the measured lead signals and wherein the measured lead vectors may be orthogonal or not orthogonal.

The problem can be broken down into a series of individual linear programs ("LPs"), which can be readily solved using available linear program solvers, by considering the solution for each row of $\mathbf{A}$.

Due to the symmetricity of the constraints about the origin, the optimization problem may optionally be further relaxed to maximizing $\mathbf{a}^T\mathbf{x}$ instead of its infinity norm, as the absolute minimum value is equal to the absolute maximum value and the two values occur at $\mathbf{x}_{max}$=- $\mathbf{x}_{min}$. Here, the optimization problem may be transformed to:

$$\text{maximize } \mathbf{a}_n^T \cdot \mathbf{x}$$

and under the constraints:

$$\|\mathbf{x}\|_1 \leq 2$$

$$\mathbf{1}^T \cdot \mathbf{x} = 0$$

where $\mathbf{a}_n$ is the vector of measured ECG lead signal values $\mathbf{a}(n)$ for sample point, $n$.

The L1-norm constraint may be decomposed into $2^{m+1}$ linear inequality constraints, with m being the number of elements of $\mathbf{x}$. The result is a linear program, which is a problem class with existing solvers and a subtype of the superset of convex optimization problems.

For Method 1, the problem above can be solved for each received vector $\mathbf{a}_n$ of measured lead signal samples. Its solution does not depend on earlier or later samples, so the method may comprise recording the identified optimal set of coefficients $\mathbf{x}_{op}$ for the synthetic lead vector and the resulting maximum value of $y_{opt}=\mathbf{a}^T\mathbf{x}$ corresponding to each time index $n$ for future use.

The problem can be solved with existing algorithms for solving linear programs and their implementations, such as simplex algorithms and inner-point algorithms.

For Method 2, the optimization problem may optionally be modified. The sample value vector $\mathbf{a_n}$ is now a vector of measured ECG lead vector samples (e.g. standard measured ECG lead vectors), and $\mathbf{x}$ a vector of weighting coefficients with different constraints.

Optionally, the L2-norm equality constraint can be relaxed to an inequality constraint due to maximization and symmetricity about the origin. In other words, the optimization problem may be simplified to:

$$\text{maximize } \mathbf{a}_n^{\mathrm{T}} \cdot \mathbf{x}$$

$$\|\mathbf{x}\|_2 \leq 1$$

This is also a convex optimization problem. It has a closed-form solution in

$$\mathbf{x}_{\mathrm{opt}} = \frac{\mathbf{a}_n^{\mathrm{T}}}{\left\|\mathbf{a}_n^{\mathrm{T}}\right\|_2}$$

In the three-dimensional case, when one or more chest or posterior electrodes are connected, it may be difficult to determine a standard vector that is orthogonal to the plane that contains the limb electrodes. The most likely candidates in this case would be V1 and V2.

Regardless of the Method used to determine the optimal synthetic lead vector, the method further comprises a process of detecting pacemaker pulses in the determined (optimized) synthetic lead vector signal. This is performed for example using a pacemaker pulse detection algorithm.

In some embodiments, the pacemaker pulse detection algorithm is configured to recurrently compare samples of the synthetic lead signal against a trailing edge detection threshold and/or a leading edge detection threshold. The threshold(s) may be static or dynamic. A pacemaker pulse can in general be distinguished from a physiological pulse by the steepness of the trailing or leading edge.

By way of illustration, one way of implementing a pacemaker pulse detection algorithm will now be outlined in detail.

The pacemaker pulse detection algorithm may include compiling a running record of tuples of ($\mathbf{a_n}$, $\mathbf{x_{opt}}$, $y_{opt}$) for a window of past samples, where, for example, the window size is chosen such as to cover at least the last three milliseconds (as pacemaker pulses are up to two milliseconds in length). Here, $\mathbf{a_n}$ is a vector of measured lead signal / lead vector values for a single sample, $n$, $\mathbf{x_{opt}}$ is the determined optimal set of weightings for forming the synthetic lead signal, and $y_{opt} = \mathbf{a_n}^{\mathrm{T}}\mathbf{x_{opt}}$, i.e. the dot product of $\mathbf{a_n}$ and $\mathbf{x_{opt}}$, i.e. the value of the synthetic lead signal for the sample point, n.

For each new sample point (i.e. time index) $n$, the method may comprise comparing $y_{opt}$ to a trailing edge detection threshold which may be static or dynamic. If $y_{opt}$ is less than the threshold, the current set of input samples $\mathbf{a_n}$ does not contain a potential leading or trailing edge of a pacemaker pulse and the pulse detection algorithm can pass to the next set of samples. If $y_{opt}$ is at or above the threshold, the pulse detection algorithm checks the set of past tuples ($n-k$, $k>0$) for $y_{opt}$ values that are at or above the leading edge detection threshold. For any such tuples, the algorithm checks whether the leading edge detection threshold is still reached using the $\mathbf{x_{opt}}$ of the most recent tuple, or if the most recent tuple still reaches the trailing edge detection threshold using the $\mathbf{x_{opt}}$ values of the past ($n-k$) tuple. If either of the checks is successful, then this indicates a pacemaker pulse is present. The pulse detection algorithm may also attempt threshold checks using the mean of $x_{opt}(n)$ and $x_{opt}(n-k)$.

Advantageously, embodiments of the invention can be applied to existing ECG measurement apparatuses via a simple software update, without requiring changes to the hardware. Alternatively, the solution may be integrated in a new hardware device.

The technical effect of using the invention is a pacemaker pulse detection functionality which is less sensitive to misalignment between the pacemaker pulse injection direction and the directions of the standard ECG lead vectors. The sensitivity of the pace pulse detection is also independent of user settings such as the selection of the "primary lead" for analysis. For a user of the system, pacemaker pulse detection is rendered more reliable, more consistent, and free of surprising technical or anatomical dependencies.

The invention may be used with different numbers of single-ended input channels, i.e. different numbers of measured lead signals. Preferably, at least three single-ended input channels are used, as a setup with only two electrodes may only allow for one lead vector to be formed and has no adjustable directional parameters.

Embodiments of the invention are compatible with different electrode placement protocols including non-standard electrode placements, e.g. EASI. The optimization process does not depend on any particular electrode placement.

Embodiments of the invention may be used with electrode setups that include posterior (on the back) placements of V electrodes, e.g. V7. V8 and V9.

Embodiments of the invention may be configured to either process each set of input channel samples individually, as described above, or use block-wise processing to find an optimal lead vector for each block of data. The optimization algorithm may thus be run recurrently at regular sampling intervals in some embodiments, which may be every sample point or at a sampling interval which is greater than one.

Once a pacemaker pulse is identified using the process described above, the method may further comprise determining one or more characteristics of the detected pulse as represented in one or more of the measured (e.g. standard) ECG vectors, for example by using the timestamps of the leading and trailing edge of the detected pulse to identify the pulse in the one or more measured ECG vectors. This allows for example for presenting an accurate annotation and reproduction of the pulse amplitude as seen in the standard lead vectors.

According to some embodiments, the method may include tracking functionality for tracking different pace pulse injection directions to better work with pacemakers that emit multiple pulses across different parts of the heart during one heart cycle, e.g. separate pulses for atria and ventricles.

Various optional variations on the above-described method(s) will now be discussed which may be applied according to one or more embodiments of the invention.

According to one or more embodiments, the cost function (objective function) utilized in the optimization may be further refined and the constraints of the optimization problem further refined.

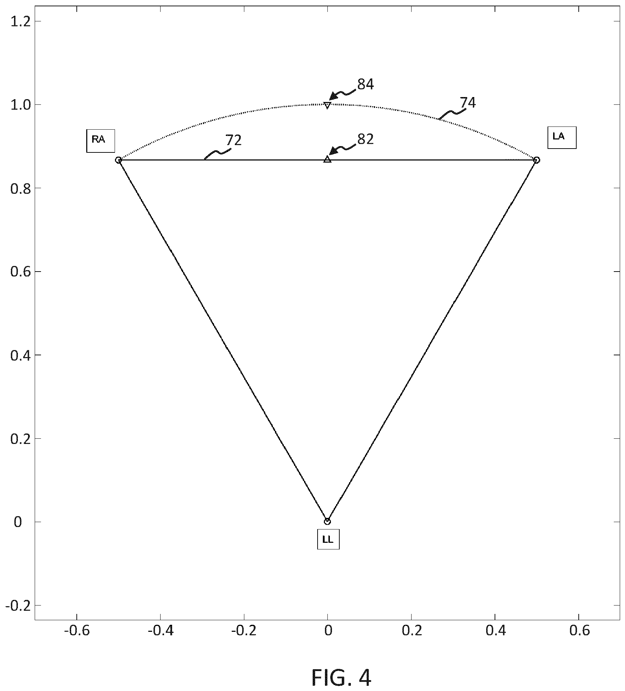

To explain: one way of defining a synthetic lead vector is to consider placement of virtual (interpolated) electrodes at locations on lines between real (measured) ECG lead electrode locations. This is illustrated in Fig. 4. This schematically shows positions of the RA, LA and LL electrodes of an ECG system.

In one case, a virtual electrode could be virtually placed at a point along a straight line between two measured ECG electrode locations. One example is shown in Fig. 4. A straight line 72 between the RA and LA electrodes is indicated. A first virtual electrode 82 is shown placed at a virtual location midway between RA and LA along the line 72. In this case, the resulting optimal synthetic lead vectors will commonly have one element that is 1, one element that is -1, and the rest of the elements being zero (a vector containing a virtual electrode will only be as optimal as, but not better than, a vector containing only real electrodes due to the properties of linear programs).

Alternatively, one could instead consider placement of virtual electrodes on an imaginary circle or arc connecting the locations of two or more different real (measured) ECG lead electrode locations. For example, one could consider placement of virtual electrodes on an imaginary circle or arc connecting the locations of RA, LA and LL. Fig. 4 shows an example a circle/arc 74 connecting the RA and LA electrodes. A second virtual electrode 84 is shown placed midway along the arc 74 between RA and LA. In this case for example, the constraint $\|x\|_1 = 2$ may be replaced by $\|x\|_2 = sqrt(2)$ (the constraint $\mathbf{1}^T\mathbf{x} = 0$ remains as it is invariant for definitions of a lead vector).

The resulting optimization program is no longer a linear program, but it remains a convex optimization problem that can be solved with existing algorithms and their implementations, such as inner-point algorithms. The constraint in the definition of a lead vector could also be replaced with an Lp norm with 1<p<2 to be closer to the original L1-norm constraint, but allow for solutions that use more than two of the single-ended input signals. In this case, the Lp norm of a lead vector is constrained to be equal to the Lp norm of a lead vector that is a difference of two input electrodes: $\|x\|_p = \|(1, -1)\|_p$. This change from the usual definition of a lead vector using the L1-norm constraint would reflect the fact that the electrical field caused by the pacemaker pulse may not be completely uniform (i.e. may not have field lines that are exactly parallel).

Optionally, the optimization problem could additionally or alternatively be further refined by extending the cost function to not only maximize a property associated with pacemaker pulses (e.g. slew rate), but concurrently optimize criteria associated with noise, or more generally, detectability of pace pulses. A lead vector that reproduces a pacemaker pulse with a slightly lower amplitude, but includes fewer undesired components which interfere with detection, may be preferable to a lead vector that reproduces the pacemaker pulse with maximal amplitude, but also contains significant undesired components.

Thus, for example in some embodiments, the objective function used in the optimization may include a pulse signal strength component correlated with an estimated strength of the pace pulse signal in the synthetic lead signal, and a noise cost component subtracted from the signal strength component, the noise cost component correlated with an estimated noise level in the synthetic lead signal.

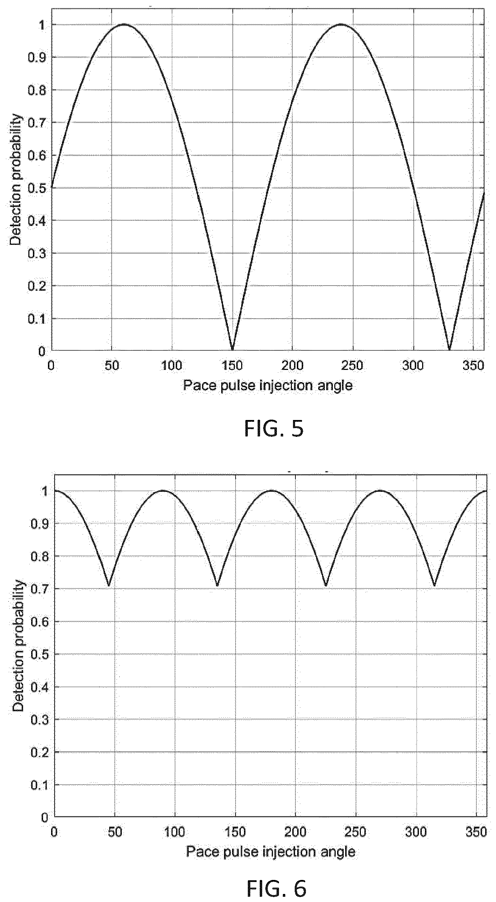

Figs. 5-8 illustrate the effect of embodiments of the present invention upon probability of detecting a pacemaker pulse in an ECG signal as compared with using standard pacemaker pulse detection methods.

In particular, these graphs show the possible resulting detection probabilities when an ECG measurement apparatus uses only one standard lead vector, two standard lead vectors, all standard lead vectors, or the optimal dynamic lead vector method described in this invention.

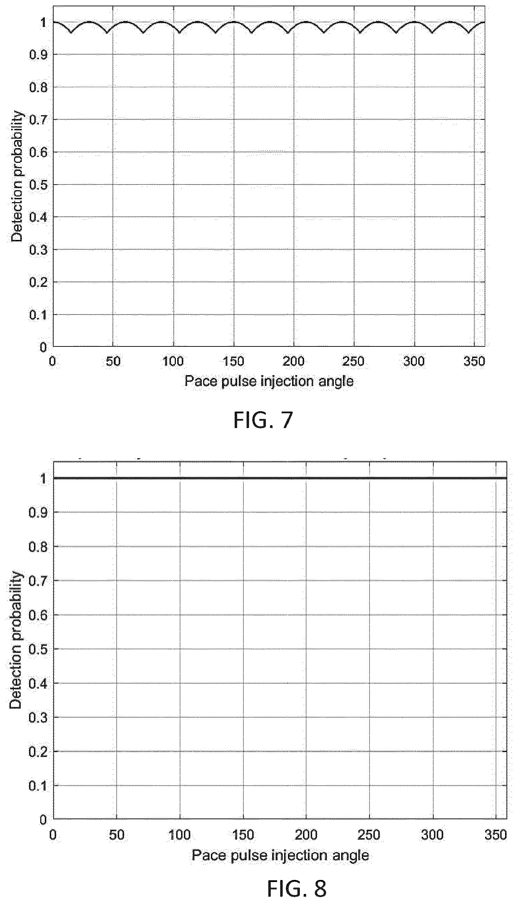

Each of Fig. 5-8 shows a graph illustrating detection probability (y-axis) as a function of pace pulse injection angle (x-axis) when using a different respective set of one or more lead signals or lead vectors to perform the detection. Figs. 5-7 show detection probabilities when using different sets of standard lead signals or vectors, while Fig. 8 shows detection probabilities when using a synthetic lead vector constructed according to the principles of the present invention. Fig. 5 shows detection probabilities when using only standard lead II for pace pulse detection. Fig. 6 shows detection probabilities when using only leads I and aVF for pave pulse detection. Fig. 7 shows detection probabilities when using all standard limb leads for pace pulse detection. Fig. 8 shows detection probabilities when using a (optimal) synthetic lead vector constructed according to the principles of the present invention.

It will be recognized that while detection probability is variable in the cases illustrated in Figs. 5-7, in the case shown in Fig. 8, detection probability is consistently high, regardless of the pace pulse injection angle.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method (10) for detecting pacemaker pulses in electrocardiogram, ECG, data comprising:

   receiving (12) ECG data comprising a plurality of recorded ECG lead signals, each recorded lead signal corresponding to a recording for a respective ECG lead of an ECG measurement apparatus;
   constructing (14) a synthetic lead signal from a weighted sum of at least a subset of the recorded lead signals and/or one or more lead vectors computed from at least a subset of the recorded ECG lead signals, wherein a lead vector comprises a linear combination of two or more of the recorded lead signals; and
   applying (20) a pacemaker pulse signal detection algorithm to the synthetic pulse signal to detect and extract a pacemaker pulse signal from the synthetic lead signal,
   wherein the constructing a synthetic lead signal comprises running an optimization procedure (16) comprising identifying a set of weightings for the weighted sum which maximizes a value of a pre-defined objective function, wherein the pre-defined objective function is correlated with a strength of the pacemaker pulse signal in the synthetic lead signal.

2. The method of claim 1, wherein the objective function includes at least a component which evaluates a maximum slew rate of the synthetic lead signal.

3. The method of claim 1 or 2, wherein the objective function, when applied to a synthetic lead signal, results in:

high pass filtering the synthetic lead signal or numerically differentiating the synthetic lead signal to yield a processed synthetic lead signal; and
subsequently computing a L-infinity norm of the processed synthetic lead signal.

4. The method of any preceding claim, wherein the optimization procedure is performed under a constraint that a sum of the weightings of the weighted sum is zero.

5. The method of any preceding claim, wherein the optimization procedure is performed under a constraint that a sum of absolute values (L1-norm) of the weightings is less than or equal to two.

6. The method of claim 5, wherein the optimization procedure is performed under a constraint that a sum of absolute values (L1-norm) of the weightings is equal to two.

7. The method of any preceding claim, wherein the objective function includes a pulse signal strength component correlated with an estimated strength of the pace pulse signal in the synthetic lead signal, and a noise cost component subtracted from the signal strength component, the noise cost component correlated with an estimated noise level in the synthetic lead signal.

8. The method of any preceding claim, wherein the synthetic lead signal is constructed only from a weighted combination of the recorded lead signals.

9. The method of any of claims 1-7, wherein the method comprises computing from the recorded ECG lead signals a first lead vector and a second lead vector, and wherein the synthetic lead signal is constructed from a weighted combination of at least the first and second lead vectors.

10. The method of claim 9, wherein the first and second lead vectors are orthogonal in directionality.

11. The method of any preceding claim, wherein the pacemaker pulse detection algorithm comprises recurrently comparing samples of the synthetic lead signal against a trailing edge detection threshold and/or a leading edge detection threshold.

12. A computer program product comprising computer program code configured, when run on a processor, to cause the processor to perform a method in accordance with any of claims 1-11.

13. A processing device, comprising one or more processors configured to perform a method in accordance with any of claims 1-11.

14. A system comprising:

a processing device in accordance with claim 13; and
an ECG apparatus operatively coupled with the processing device.

EP 4 640 152 A1

FIG. 1

15

FIG. 2

FIG. 3

FIG. 4

FIG. 5

FIG. 6

FIG. 7

FIG. 8

## EUROPEAN SEARCH REPORT

Application Number

EP 24 17 2484

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 581 073 A2 (SPACELABS MEDICAL INC [US]) 2 February 1994 (1994-02-02) * page 4, line 24 - page 9, line 50 * * page 12, line 13 - page 13, line 54 * * figures * | 1-14 | INV. A61B5/327 A61B5/00 |
| A | MCGLINCHEY G ET AL: "A PROGRAMMABLE MEDICAL DATA ACQUISITION SYSTEM CHIP", PROCEEDINGS OF THE CUSTOM INTEGRATED CIRCUITS CONFERENCE. NEW YORK, MAY 16 - 19, 1988; [PROCEEDINGS OF THE CUSTOM INTEGRATED CIRCUITS CONFERENCE], NEW YORK, IEEE, US, vol. CONF. 10, 16 May 1988 (1988-05-16), pages 941-946, XP000124779, * page 942, left-hand column, paragraph 3 - page 946, left-hand column, paragraph 1 * * figures * | 1-14 | |
| A | EP 1 038 498 A2 (ST GEORGE S ENTERPRISES LTD [GB]) 27 September 2000 (2000-09-27) * paragraph [0017] - paragraph [0019] * * paragraph [0107] - paragraph [0122] * * paragraph [0149] - paragraph [0167] * | 1-14 | TECHNICAL FIELDS SEARCHED (IPC) A61B |
| A | WO 92/02171 A1 (SPACELABS INC [US]) 20 February 1992 (1992-02-20) * page 3, line 26 - page 8, line 27 * * figures 1-9 * | 1-14 | |

The present search report has been drawn up for all claims

| Place of search | Date of completion of the search | Examiner |
|---|---|---|
| The Hague | 20 September 2024 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 2484

This annex lists the patent family members relating to the patent documents cited in  the above-mentioned European search report.
The members are as contained in the European Patent Office EDP file on
The European Patent Office is in no way liable for these particulars which are merely given for the purpose of information.

20-09-2024

| Patent document cited in search report | | Publication date | Patent family member(s) | | Publication date |
|---|---|---|---|---|---|
| EP 0581073 | A2 | 02-02-1994 | CA | 2098977 A1 | 10-01-1994 |
| | | | EP | 0581073 A2 | 02-02-1994 |
| | | | US | 5231990 A | 03-08-1993 |
| EP 1038498 | A2 | 27-09-2000 | EP | 1038498 A2 | 27-09-2000 |
| | | | US | 6438409 B1 | 20-08-2002 |
| WO 9202171 | A1 | 20-02-1992 | CA | 2067225 A1 | 04-02-1992 |
| | | | EP | 0495081 A1 | 22-07-1992 |
| | | | US | 5058598 A | 22-10-1991 |
| | | | WO | 9202171 A1 | 20-02-1992 |

EPO FORM P0459

For more details about this annex : see Official Journal of the European Patent Office, No. 12/82